# EUROPÄISCHE PATENTSCHRIFT

(11) **EP 2 705 358 B1**
(45) Veröffentlichungstag und Bekanntmachung des Hinweises auf die Patenterteilung: **22.02.2017**
(21) Anmeldenummer: 12730165.3
(22) Anmeldetag: 29.05.2012
(51) Int. Cl.: G01N 27/414, G01N 33/00

(54) **VORRICHTUNG UND VERFAHREN ZUR ERMITTLUNG DES KOHLENDIOXIDGEHALTS VON LUFT**
DEVICE AND METHOD FOR DETERMINING THE CARBON DIOXIDE CONTENT OF AIR
DISPOSITIF ET PROCÉDÉ POUR LA DÉTERMINATION DE LA TENEUR EN DIOXYDE DE CARBONE DE L'AIR

(30) Priorität: 15.06.2011 DE 102011077559
(43) Veröffentlichungstag der Anmeldung: 12.03.2014
(73) Patentinhaber: Siemens Aktiengesellschaft, 80333 München (DE)
(72) Erfinder: FLEISCHER, Maximilian, 85635 Höhenkirchen (DE); POHLE, Roland, 85570 Herdweg (DE); STEGMEIER, Stefan, 81541 München (DE); VON SICARD, Oliver, 80469 München (DE)
(86) Internationale Anmeldenummer: PCT/EP2012/060001
(87) Internationale Veröffentlichungsnummer: WO 2012/171783

(56) Entgegenhaltungen:
- EP-A1- 1 510 814
- EP-A2- 1 176 418
- WO-A1-97/28441
- H.-E. ENDRES ET AL.: "A capacitive C02 sensor system with suppression of the humidity interference", SENSORS AND ACTUATORS B, Bd. 57, 1999, Seiten 83-87, XP002681276, in der Anmeldung erwähnt
- STEGMEIER S ET AL: "Sensing of COat room temperature using work function readout of (hetero-)polysiloxanes sensing layers", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, Bd. 154, Nr. 2, 11. Januar 2010 (2010-01-11), Seiten 206-212, XP028383721, ISSN: 0925-4005, DOI: 10.1016/J.SNB.2009.12.071 [gefunden am 2010-01-11]

## Beschreibung

Die Erfindung betrifft einen Kohlendioxidsensor zur Ermittlung des Kohlendioxidgehalts von Luft. Weiterhin betrifft die Erfindung ein Verfahren zur Erzeugung eines Gasmesswerts, der die Kohlendioxidkonzentration in Luft repräsentiert.

Die Detektion von Kohlendioxid ist für eine Reihe von Applikationen von hohem Interesse. Beispiele sind die Beurteilung der Luftgüte in Innenräumen, energieeffizientes Ansteuern von Klimaanlagen oder die Kontrolle gereinigter Luft. Ziel der Detektion von Kohlendioxid kann eine Erhöhung des Komforts sein. Es ist aber auch möglich, unter Umständen erhebliche Energieeinsparungen zu erzielen.

So kann beispielsweise bei einem gut isolierten Gebäude nahezu die Hälfte der für eine Klimatisierung benötigten Energie durch eine bedarfsgerechte Klimatisierung eingespart werden. Der Bedarf orientiert sich dabei unter anderem am Kohlendioxid-Gehalt der Luft. Auch im Automobilbereich ist eine bedarfsgerechte Belüftung und Klimatisierung des Fahrgastinnenraums vorteilhaft. Ein Schätzwert für die Reduzierung des Verbrauchs für die Klimatisierung beträgt 0,3 l auf 100 km.

Kohlendioxid tritt bei normalen Umgebungsbedingungen in der Luft in einer Konzentration von ca. 380-400 ppm auf. Ein Sensor für Kohlendioxid muss ausgehend von dieser Basiskonzentration in der Lage sein, erhöhte Konzentrationen bis beispielsweise 4000 ppm zu detektieren. Problematisch ist dabei, dass das Kohlendioxidmolekül ein lineares, symmetrisches Molekül ist und daher kein elektrisches Dipolmoment vorhanden ist, welches bei verschiedenen Transducer-Prinzipien ein Sensorsignal bewirken kann. Weiterhin ist das Molekül chemisch sehr unreaktiv.

Momentan sehr erfolgreiche Methoden zur Konzentrationsbestimmung von Kohlendioxid sind daher vor allem im Bereich der optischen Spektroskopie zu finden. Hierbei wird ausgenutzt, dass Kohlendioxid in bestimmten Wellenlängenbereichen, beispielsweise bei etwa 4,3 µm Wellenlänge, Licht absorbiert. Hierdurch ist eine genaue und selektive Messung der Konzentration von Kohlendioxid möglich. Dabei kommt es auf die chemische Reaktivität des Kohlendioxids nicht an. Nachteilig an der optischen Spektroskopie sind jedoch der komplexe Aufbau der Messsysteme und der erhebliche Aufwand, der zur Auswertung der gemessenen Spektren erforderlich ist. Das führt letztlich zu verhältnismäßig großen und teuren Messsystemen.

Festkörpersensoren wie beispielsweise Halbleiter-Gassensoren vermeiden die Nachteile der optischen Messsysteme. Sie sind klein, durch Massenproduktion im Vergleich extrem billig herzustellen und benötigen eine weniger komplexe Signalauswertung. Nachteilig bei Festkörpersensoren ist jedoch, dass sie auf eine gewisse Reaktivität der zu messenden Moleküle angewiesen sind und gleichzeitig aber alle Moleküle detektieren, die eben eine gewisse Reaktivität aufweisen. Anders formuliert haben die Festkörpersensoren eine geringe Selektivität. Das macht vor allem die Messung wenig reaktiver Spezies wie Kohlendioxid mit solchen Sensoren schwierig, da sie meist sehr stark auf Kohlenwasserstoffe oder Ozon reagieren.

Die Reihe der potentiellen Störgase ist dabei umfangreich. Sie umfasst Stickstoffdioxid (NO₂), Kohlenmonoxid (CO) und Wasserstoff (H₂), Ammoniak (NH₃), Ethanol oder Salzsäuren (HCl), Stickstoffmonoxid (NO), Schwefeloxide (SOₓ), Kohlenoxidsulfid (COS), Lachgas (N₂O) und Blausäure (HCN), Wasser (H₂O) sowie organische Gase wie Methan, Ethan, Ethen, Acetylen und andere Kohlenwasserstoffe wie Formaldehyd (CH₂O). Weitere Störgase sind Amine (NH₂R₁, NH₁R₂, N_{R}3), Amide (RC(O)NH, RC(O)NHR', RC(O)NR'R), Acrolein (C₃H₄O) und Phosgen (COCl₂), Aromate wie Benzol (C₆H₆), Ethylbenzol, Chlorbenzol, Toluol, Xylol, Styrol und Phenol (C₆H₆O). Des Weiteren gibt es Ozon (O₃), die große Gruppe der VOCs (volatile organic compounds).

Diese Gase treten teilweise schon in der normalen Umgebungsluft auf, beispielsweise Ozon. Weitere Quellen für Gase sind Brände, Zigarettenrauch, menschliche Aktivität, die Verwendung chemischer Mittel wie Putzmittel, offenstehende Nahrungsmittel oder technische Geräte wie Drucker. Auch Straßenverkehr und sogar die Wetterverhältnisse führen zum Auftreten von Gasen.

Aus der Schrift von H.-E. Endres et al., "A capacitive CO2 sensor system with suppression of the humidity interference", Sensors and Actuators B 57 (1999), 83-87 ist ein CO₂-Sensor bekannt, der auf dem Prinzip einer Kapazitätsmessung beruht. Bei dem offenbarten kapazitiven Sensor wird ein zusätzlicher Feuchtesensor verwendet, um ein Feuchtesignal zu erzeugen.

Ein potentialgesteuerter Feuchtesensor, der hierfür verwendbar ist, ist beispielsweise aus der EP 1 176 418 A2 bekannt. Der potentialgesteuerte Feuchtesensor weist einen gassensitiven Bereich auf, der unabhängig von einer Feuchte polarisierbar ist. Weiterhin weist der gassensitive Bereich eine relative Dielektrizitätskonstante auf, die von der Feuchte abhängig ist.

Ein weiterer CO₂-Sensor ist auch aus WO97/28441 bekannt. Der Sensor wird bei zwei unterschiedlichen Temperaturen betrieben (z.B. 300 °C und 500 °C). Dabei werden bei der höheren Temperatur adsorbiertes Wasser und andere interferierende Stoffe vom Sensor entfernt, um bei der niedrigeren Temperatur einen unverfälschten Messwert zu erhalten.

Nachteilig an den bekannten Sensoren ist, dass zur Ermittlung und Kompensation der Luftfeuchte ein weiterer Sensor erforderlich ist. Dieser muss, ebenso wie der eigentliche Kohlendioxidsensor, kalibriert und ausgewertet werden und muss die für den Kohlendioxidsensor vorausgesetzten Eigenschaften, beispielsweise der Langlebigkeit, miterfüllen.

Es ist Aufgabe der vorliegenden Erfindung, einen Gassensor anzugeben, der eine Detektion von Kohlendioxid ermöglicht. Eine weitere Aufgabe besteht darin, ein Verfahren zur Erzeugung eines Gasmesswerts, der die Kohlendioxidkonzentration in Luft repräsentiert, anzugeben. Dabei soll jeweils insbesondere der Einfluss von Umgebungsvariablen auf das Messsignal in ausreichender Weise kompensiert werden. Diese Aufgabe wird durch eine Vorrichtung mit den Merkmalen von Anspruch 1 gelöst. Die Aufgabe wird weiterhin durch ein Verfahren mit den Merkmalen von Anspruch 13 gelöst. Die abhängigen Ansprüche betreffen vorteilhafte Ausgestaltungen der Erfindung.

Die erfindungsgemäße Vorrichtung zur Ermittlung des Kohlendioxidgehalts von Luft weist wenigstens einen kohlendioxidsensitiven Gassensor zur Ausgabe eines Gasmesswerts auf. Weiterhin weist sie eine Einrichtung zur Temperatursteuerung des Gassensors auf. Weiterhin ist eine Einrichtung zur Auswertung des Gasmesswerts zur Ermittlung des Kohlendioxidgehalts der Luft vorgesehen.

Die Vorrichtung ist derart ausgestaltet, dass die Einrichtung zur Temperatursteuerung im Betrieb des Gassensors wenigstens zwei verschiedene Temperaturen einstellt und bei jeder der zwei verschiedenen Temperaturen wenigstens ein Gasmesswert aufgezeichnet wird.

Ferner ist die Vorrichtung derart ausgestaltet, dass die Einrichtung zur Auswertung aus den Gasmesswerten bei den verschiedenen Temperaturen den Kohlendioxidgehalt unter Korrektur des Einflusses der Luftfeuchte ermittelt, indem die Einrichtung zur Auswertung die Gasmesswerte unter der Voraussetzung miteinander verrechnet, dass die Sensitivität des Gassensors auf Kohlendioxid einen anderen Verlauf mit der Temperatur des Gassensors aufweist als die Sensitivität auf Wasser.

Der Gassensor weist dabei ein gassensitives Material auf, beispielsweise in Form einer gassensitiven Schicht. Das gas-sensitive Material ist so gestaltet, dass es auf Kohlendioxid anspricht. Mit anderen Worten ändert sich der Gasmesswert bei einer Änderung der Konzentration von Kohlendioxid in der umgebenden Luft. Dabei ist es zweckmäßig, wenn diese Änderung bei einer Konzentrationsänderung von insbesondere 50 ppm CO₂ messbar ist, also die Änderung größer ist als das Signalrauschen. Es ist in einem anderen Beispiel auch ausreichend, wenn die Änderung des Gasmesswerts bei einer Konzentrationsänderung von 500 ppm CO₂ messbar ist.

Es wird hierbei von der Konzentration von Kohlendioxid bzw. Wasser in Luft gesprochen. Darunter wird auch die tatsächliche Messart der meisten gassensitiven Materialien verstanden, bei der nicht ein relativer Anteil eines Gases gemessen wird, sondern das absolute Vorhandensein, d.h. der Partialdruck des Gases. Der Gasmesswert hängt also zweckmäßig vom Partialdruck von Kohlendioxid und Wasser ab.

Insgesamt ist vorteilhaft die Vorrichtung so gestaltet, dass die Verrechnung der Gasmesswerte dazu führt, dass der Einfluss einer Änderung der Luftfeuchte auf den Gasmesswert verringert wird. Dabei wird durch die Temperatursteuerung des Gassensors allein erreicht, dass eine Korrektur des Gasmesswerts ermöglicht wird. Es ist mit anderen Worten kein zusätzlicher Feuchtesensor mit allen verbundenen Nachteilen notwendig.

Ein besonderer Vorteil besteht darin, dass gleichzeitig mit der Korrekturmöglichkeit für den Gasmesswert auch ein Ausheizen des Sensors erfolgt. D.h. Moleküle, die bei Raumtemperatur an beispielsweise der gassensitiven Schicht adsorbiert bleiben würde, werden durch den intermittierenden Betrieb bei erhöhter Temperatur entfernt, wodurch die Basislinie des Gassensorsignals insgesamt stabilisiert wird.

Gemäß einer vorteilhaften Ausgestaltung der Erfindung wird bei dem Gassensor der Gasmesswert durch eine Auswertung der Austrittsarbeit des Materials erzeugt.

Gemäß einer weiteren vorteilhaften Ausgestaltung der Erfindung weist das gassensitive Material primäre Aminogruppen (R-NH₂, R=Rest, z. B. Alkylrest) auf. Diese primären Aminogruppen bilden bei Raumtemperatur bei Vorhandensein von CO₂ reversibel geladene Spezies (z. B. Bikarbonat und Carbamat), die zu einer deutlichen Änderung der Austrittsarbeit führen. Materialien mit primären Aminogruppen zeigen eine deutliche reversible Reaktion auf Änderungen des Partialdrucks von Kohlendioxid. Gleichzeitig hat sich gezeigt, dass die Querempfindlichkeiten gegenüber gewissen Störgasen wie beispielsweise NO₂, flüchtige Kohlenwasserstoffe oder Lösemittel nicht groß sind.

Es hat sich in Messungen aber vor allem überraschend herausgestellt, dass gerade CO₂-Sensoren basierend auf Austrittsarbeitsmessung an dem Material mit primären Aminogruppen unter Umständen auf Luftfeuchte ansprechen, und zwar stärker als auf die vielen sonstigen Störgase. Dies war bereits deswegen unerwartet, weil bei vielen der üblicherweise verwendeten Schichtmaterialien für die Austrittsarbeitsmessung zwar Querempfindlichkeiten auftreten, aber gerade für Wasser kaum. Beispiele sind die Kupferphtalocyanine (CuPC), Bleiphtalocyanine (PbPC) oder sonstige Phtalocyanine, Galliumoxid (Ga₂O₃), Platin (Pt) und Titannitrid (TiN).

Für Störgase, die bei primären Aminogruppen eine geänderte Austrittarbeit auslösen, gäbe es wiederum näher liegende Kandidaten. Die Luft enthält nämlich in wechselnder Konzentration Gase, die mit primären Aminen (...-NH₂) stark reagieren, wie insbesondere Stickstoffdioxid (NO₂), Alkohole (R-OH), Stickstoffmonoxid (NO), Ozon (O₃), Wasserstoff (H₂), Kohlenmonoxid (CO), Ammoniak (NH₃), Salzsäuren (HCl), Blausäure (HCN), Schwefeloxide (SOₓ), Kohlenoxidsulfid (COS), Lachgas (N₂O) und organische Gase.

Dabei zeigt sich der Einfluss von Wasser nicht eindeutig klarer als der Einfluss anderer Quellen auf das Messergebnis. Ähnlich und nicht sofort unterschiedlich ändert sich das Messergebnis infolge von Temperaturänderungen, Verschmutzung, sowie Unvollkommenheiten in der Signalauswertung, Nullpunktsdrift und Hystereseeffekte, d.h. Vorbelastungen aus vorangehenden Messreihen. Daher blieb der überraschend festgestellte Effekt der Luftfeuchte während einer Reihe von Messungen sogar unentdeckt.

Bei dem erfindungsgemäßen Verfahren zur Ermittlung des Kohlendioxidgehalts von Luft mittels eines kohlendioxidsensitiven Gassensors unter Korrektur des Einflusses der Luftfeuchte wird im Betrieb der Gassensor auf wenigstens zwei verschiedene Temperaturen gebracht, bei jeder der zwei verschiedenen Temperaturen wenigstens ein Gasmesswert aufgezeichnet und aus den Gasmesswerten bei den verschiedenen Temperaturen der Kohlendioxidgehalt unter Korrektur des Einflusses der Luftfeuchte ermittelt, indem die Gasmesswerte unter der Voraussetzung miteinander verrechnet werden, dass die Sensitivität des Gassensors auf Kohlendioxid einen anderen Verlauf mit der Temperatur des Gassensors aufweist als die Sensitivität auf Wasser.
Somit wird der Gasmesswert so korrigiert, dass der Einfluss der Luftfeuchte auf die Kohlendioxidmessung wenigstens verringert ist.

Besonders vorteilhaft ist es, wenn die Reaktion des Gasmesswerts auf eine Änderung der relativen Luftfeuchte um 10 % wenigstens 5 %, insbesondere wenigstens 10 % oder in einem anderen Beispiel wenigstens 20 % der Stärke der Reaktion auf eine Konzentrationsänderung von Kohlendioxid um 1000ppm aufweist.

Als Aufbau für den Gassensor ist ein Feldeffekttransistoraufbau besonders vorteilhaft. Der grundsätzliche Feldeffekttransistoraufbau ist von elektronischen Komponenten her bekannt, d.h. es existieren Drain- und Source-Elektroden und ein leitender Kanal benachbart zu einer Gate-Elektrode. Das besondere am Gassensor mit Feldeffekttransistoraufbau ist, dass das gassensitive Material benachbart zum leitenden Kanal vorgesehen ist. Dadurch beeinflussen elektrische Änderungen im gassensitiven Material die Leitfähigkeit im Kanal.

Die Funktionsweise von Gassensoren auf der Basis von Austrittsarbeitsänderungen bzw. Kontaktpotentialmessungen, wie beispielsweise mittels eines gassensitiven Feldeffekttransistors, beruht auf der physikalischen Tatsache, dass adsorbierte Gasmoleküle an der Materialoberfläche entweder als permanente Dipole vorliegen oder Dipole induzieren. Die Austrittsarbeit des mit Gas bedeckten Materials ändert sich dann um den Potentialsprung an der Dipolschicht auf der Oberfläche. Diese Potentialänderung kann in die Gatespannung eines Feldeffekttransistors eingekoppelt werden, wobei dann als Messgröße die Änderung der Einsatzspannung bei konstantem Strom verwendet werden kann.

Ein solcher Feldeffekttransistoraufbau lässt sich realisieren, indem das gassensitive Material direkt auf die Gate-Elektrode aufgebracht wird. In diesem Fall ist die Herstellung des Sensors in großen Mengen in der mikromechanischen Fertigung möglich. Dabei kann es vorteilhaft sein, wenn das gassensitive Material entweder sehr dünn gemacht wird, oder aber gasdurchlässig gestaltet wird, um eine möglichst große elektrische Wirkung der Gasreaktionen auf den leitenden Kanal zu erzielen.

Besonders vorteilhaft ist der GasFET mit einem Luftspalt zwischen der sensitiven Schicht und dem leitenden Kanal des Feldeffekttransistoraufbaus ausgestattet. Die Realisierung eines Gas-Feldeffekttransistors (GasFET), bei dem zwischen Gate-Elektrode und Kanalbereich des Transistors ein kleiner Luftspalt (0,5 - 5 µm) vorhanden ist, sieht vor, dass die dem Luftspalt zugewandte Seite der Gate-Elektrode mit dem gassensitiven Material versehen, beispielsweise beschichtet, ist. An der sensitiven Schicht entsteht durch die gasinduzierte Änderung der Elektronenaustrittsarbeit ein zusätzliches Potential in der Größenordung von typischerweise 10-100 mV, welches als zusätzliche Gatespannung auf den Transistor wirkt.

Ein besonders vorteilhafter Aufbau sieht einen GasFET, insbesondere einen hybriden GasFET mit einer gassensitiven Schicht mit primären Aminogruppen vor. Vorteilhaft ist es weiterhin, wenn das gassensitive Material ein Polymer aufweist. Die Stärke der Querempfindlichkeit zur Luftfeuchte von polymerbasierten gassensitiven Schichten hängt von der Art der Auslesung des Signals ab. Gerade beim Übergang von bekannten kapazitiven Sensoren zur Auslesung mittels der Austrittsarbeit, insbesondere mittels eines GasFETs, ergeben sich neben dem überraschenden und erheblichen Vorteil der Messung bei Raumtemperatur auch veränderte Querempfindlichkeiten. So ist die Empfindlichkeit auf Luftfeuchte überraschend stark bei einer Auslesung mittels Austrittsarbeit, wie sie bei GasFETs vorgenommen wird.

In einer besonders vorteilhaften Ausgestaltung und Weiterbildung der Erfindung ist die Vorrichtung derart ausgestaltet, dass eine der verschiedenen Temperaturen eine Temperatur ist, bei der das gassensitive Material nicht mehr auf Kohlendioxid reagiert. Mit anderen Worten wird eine Temperatur gewählt als einer der verschiedenen Temperaturen, bei der die Sensitivität des Gassensors auf Kohlendioxid wenigstens weitgehend verschwindet. Vorteilhaft ist dann die Korrektur des Gasmesswerts besonders einfach, da der Gasmesswert bei dieser Temperatur praktisch nur einer Feuchtemessung entspricht.

Um Einflüsse des Drucks ausgleichen zu können, ist in einer weiteren Ausgestaltung der Erfindung ein Drucksensor vorgesehen. Der Luftdruck kann beispielsweise durch die Höhe über dem Meeresspiegel und durch Wettereinflüsse variieren. Besonders vorteilhaft ist es, wenn der Drucksensor monolithisch mit dem Gassensor und/oder den weiteren vorhandenen Sensoren realisiert ist, d.h. auf einem gemeinsamen Substrat erzeugt.

Der Einfluss der Luftfeuchte auf den Gasmesswert lässt sich in 2 Wirkungen aufteilen. Die erste Wirkung entspricht der Funktion, die das gassensitive Material als Feuchtesensor haben könnte. Sie ist völlig unabhängig vom Vorhandensein von Kohlendioxid.

Die zweite Wirkung besteht darin, dass die Größe des Messeffekts auf CO₂ etwas von der jeweils herrschenden Luftfeuchtigkeit abhängt, d.h. das durch beispielsweise eine Konzentrationsänderung von 100 ppm Kohlendioxid hervorgerufene Signal ist abhängig vom Wasserpartialdruck. So kann eine Konzentrationsänderung von 100 ppm Kohlendioxid bei einer Luftfeuchte von 30 % beispielsweise eine Änderung des elektrischen Signals von 10 mV erzeugen, während die gleiche Konzentrationsänderung bei 70 % Luftfeuchte eine Änderung des elektrischen Signals von 13 mV erzeugt. Dabei ist der Unterschied von 40 % Luftfeuchte, der selbst beispielsweise eine Signaländerung von 40 mV erzeugt, bereits berücksichtigt und korrigiert. D.h., selbst, wenn die erste Wirkung, die weiter oben erläutert ist, vollständig korrigiert wird, verbleibt die zweite Wirkung und kann ebenfalls korrigiert werden, um die Genauigkeit der Kohlendioxid-Messung zu erhöhen.

In einer vorteilhaften Ausgestaltung der Erfindung wird die erste Wirkung korrigiert. Besonders vorteilhaft für die Genauigkeit der Messung ist es, wenn zusätzlich die zweite Wirkung korrigiert wird. Dabei wird also nicht nur der durch Feuchte bewirkte Ausschlag des Gasmesswerts herausgerechnet, sondern zusätzlich der übrigbleibende Gasmesswert, der dann vom Kohlendioxid stammt, anhand der Feuchte korrigiert.

Hierzu kann aus den Gasmesswerten bei den verschiedenen Temperaturen auch die Luftfeuchte bestimmt werden. Dann wird beispielsweise anhand von tabellarisch vorhandenen Korrekturwerten der Gasmesswert nachkorrigiert. Es ist auch möglich, die Korrektur analytisch mit hinterlegten Koeffizienten durchzuführen.

In einer vorteilhaften Ausgestaltung der Erfindung ist die Vorrichtung so gestaltet, dass die Verweildauer bei der höheren der verschiedenen Temperaturen höchstens der Hälfte der Verweildauer bei der geringeren der verschiedenen Temperaturen entspricht. Mit anderen Worten wird der Gassensor nur für eine kürzere Zeit bei erhöhter Temperatur betrieben im Vergleich zum Betrieb bei beispielsweise Raumtemperatur. Hierdurch wird vorteilhaft Energie gespart. Insbesondere kann die Verweildauer bei der höheren der verschiedenen Temperaturen nur ein Zehntel der Verweildauer geringeren der verschiedenen Temperaturen betragen.

In einer vorteilhaften Ausgestaltung der Erfindung ist die Vorrichtung ausgestaltet, die Einstellung der verschiedenen Temperaturen kontinuierlich vorzunehmen, insbesondere indem Temperaturrampen oder sinusartige Temperaturverläufe verwendet werden. Beispielsweise kann also die Temperatur in kontinuierlichen Dreiecks- oder Sägezahnrampen geändert werden.

Dabei ist es vorteilhaft, wenn neben der beschriebenen Auswertung der Gasmesswerte bei den verschiedenen Temperaturen eine Auswertung des Zeitverlaufs der Signale erfolgt, beispielsweise. mit multivariaten Verfahren wie PLS.
- Figur 1: einen Aufbau für einen Kohlendioxidsensor als SGFET,
- Figur 2: ein Messergebnis einer AMO/PTMS-Schicht für Kohlendioxid,
- Figur 3: ein Messergebnis einer AMO/PTMS-Schicht für CO₂ und Wasser,

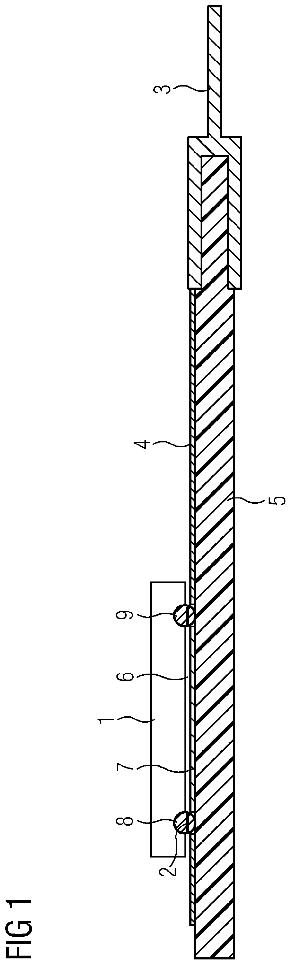

Die Fig. 1 zeigt den grundsätzlichen Aufbau eines gassensitiven FETs gemäß einem Beispiel für einen erfindungsgemäßen Aufbau. Diese umfasst einen CMOS-Transistor 1 mit einer Source-Elektrode 8 und einer Drain-Elektrode 9. Dabei wird eine FET-Struktur in Form des CMOS-Transistors 1 in Flip-Chip-Technik auf ein mit Leiterbahnen 4 versehenes Keramiksubstrat 5 montiert. Dies kann beispielsweise mittels eines Leitklebstoffes 2 geschehen. Die gassensitive Schicht 7 ist partiell auf dem Keramiksubstrat 5 aufgebracht und mit den Leiterbahnen 4 entsprechend kontaktiert. Der Gaskanal ist der Luftspalt 6 zwischen Gate und CMOS-Transistor. Das Keramiksubstrat 5 dient als Träger der gassensitiven Schicht und gleichzeitig als Träger des gesamten Sensoraufbaus, sodass in diesem Beispiel kein Einbau in einen Sensorsockel notwendig ist. Auf dieses Keramiksubstrat 5 können Steckstifte 3 angebracht werden, sodass das elektronische Bauelement direkt beispielsweise in eine Singleinline-Steckverbindung eingebracht werden kann. Alternativ sind auch andere Ausführungen möglich, beispielsweise die Ausführung als SMD-Bauelement (surface mounted device).

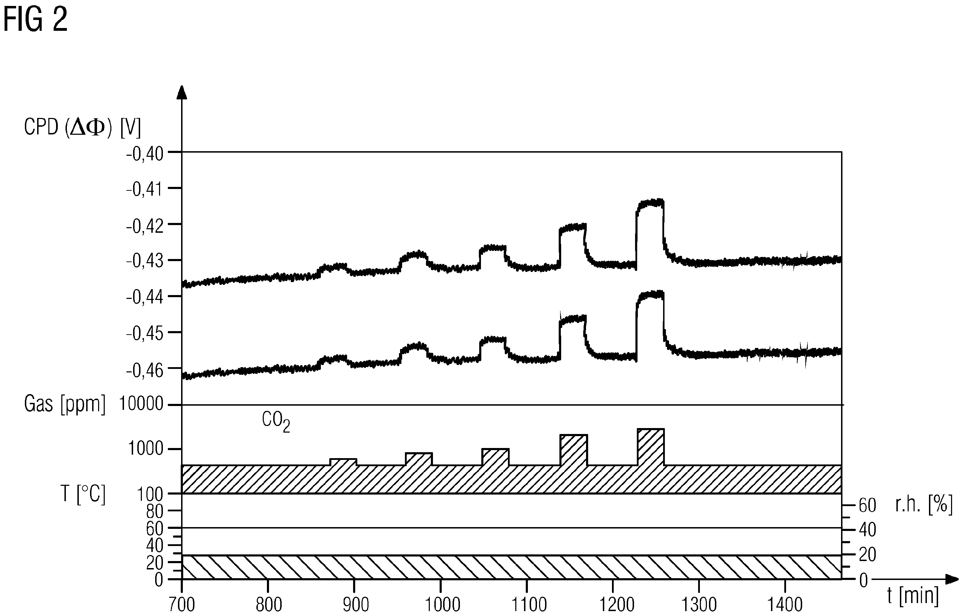

Ein erster Sensor, dessen Messergebnis in Figur 2 dargestellt ist, weist eine sog. AMO/PTMS-Schicht als Sensorschicht auf. Dieses Materialsystem wird auch als Heteropolysiloxan bezeichnet, da hier das Material aus zwei verschiedenen Ausgangssilanen gebildet wird. Zur Herstellung dieser Schicht wird Aminopropyltrimethoxysilan (AMO) und Propyltrimethoxysilan (PTMS) in Methanol gelöst. Die Lösung wird in einem Glaskolben unter Zusatz einer geringen Menge Wasser 3 Stunden unter Rückfluss gekocht. Die entstehende Lösung wird nach dem Abkühlen mittels eines Spin-Coating-Prozesses auf ein Substrat (z. B. mit Gold beschichtetes Al₂O₃-Keramik) aufgebracht und im Ofen in Stickstoffatmosphäre bei 120°C sechzehn Stunden lang ausgehärtet. Die so erzeugte Schicht weist in diesem Beispiel eine Dicke von 12,8 µm auf.

Figur 2 zeigt zwei Messergebnisse an der so erhaltenen Sensorschicht mittels einer Kelvinsonde. Während der Messdauer wurde der erste Sensor auf Raumtemperatur betrieben, also ohne Beheizung. Die künstlich erzeugte Gasumgebung der Sensorschicht wies eine relative Feuchte von 40 % auf. Während der mehrstündigen Messung wurde die Konzentration von Kohlendioxid von einem Grundniveau von ca. 400 ppm in Intervallen stufenweise angehoben und wieder auf das Grundniveau zurückgesetzt. Die kleinste erzeugte erhöhte Konzentration lag bei ca. 600 ppm, also ca. 200 ppm über dem Grundniveau. Die höchste erzeugte Konzentration lag dabei bei ca. 4000 ppm. Das Messsignal CPD (contact potential difference) zeigt einen deutlichen Ausschlag bei einer Konzentration von 4000 ppm CO₂. Bei geringeren Konzentrationserhöhungen ist das Signal entsprechend schwächer. Auch bei der geringsten Konzentrationserhöhung von ca. 200 ppm ist das Signal deutlich erkennbar.

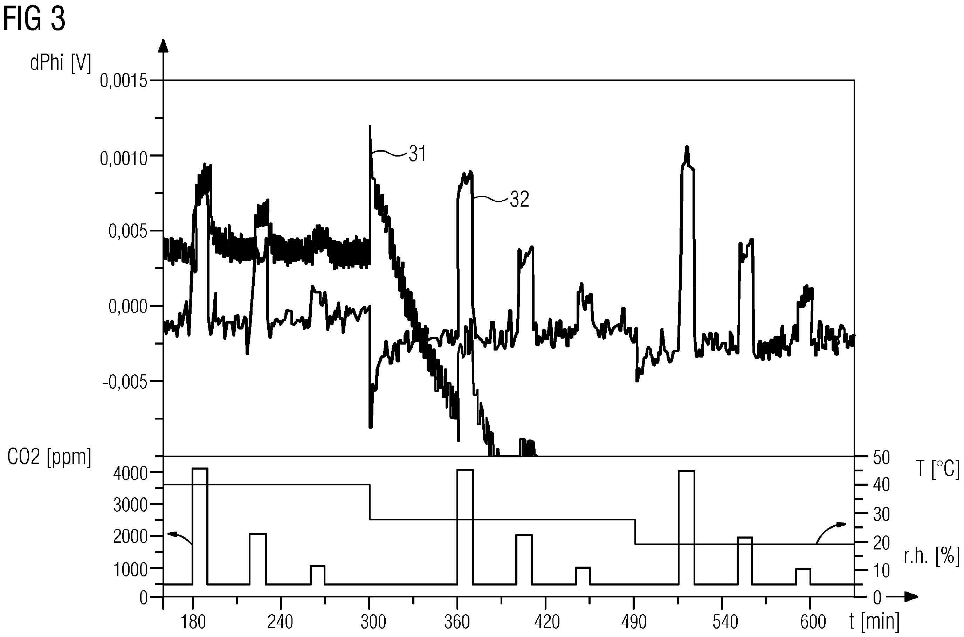

Figur 3 zeigt zwei Messergebnisse an einer AMO-Sensorschicht. Der Aufbau des Sensors ist in Figur 1 gezeigt. Die künstlich erzeugte Gasumgebung der Sensorschicht wurde so gesteuert, dass sowohl die Feuchte als auch die Kohlendioxidkonzentration variiert wurde. Ein erstes Messergebnis 31 zeigt dabei den Verlauf des Sensorsignals ohne Temperaturvariation. Es ist erkennbar, dass die Sensorschicht deutliche Reaktionen auf Änderungen der Feuchtigkeit zeigt, insbesondere eine Signaldrift bei der Änderung der Luftfeuchte.

Ein zweites Messergebnis 32 zeigt den Verlauf des korrigierten Sensorsignals, wobei eine Messung bei verschiedenen Temperaturen vorgenommen wurde. Es zeigt sich, dass die Signaldrift bei Änderungen der Feuchte deutlich reduziert ist, d.h. der Einfluss der Feuchte ist deutlich reduziert. Bei dem beispielhaften Sensor wurde als höhere Temperatur eine Temperatur von 65 °C gewählt. Bei dem konkreten Sensor, der verwendet wurde, verschwindet die Sensitivität auf Kohlendioxid bei dieser Temperatur fast völlig. Die niedrigere Temperatur war 30 °C. Da die Sensitivität auf Kohlendioxid bei 65 °C vernachlässigbar war, ergab sich als Korrekturformel für den Kohlendioxidmesswert dPhi:
30°C: dPhi = L([H₂O], [CO₂])
65°C: dPhi = L([H₂O])
-> dPhi (30°C) - A * dPhi (65°C) - L([CO₂])

Dabei bezeichnet L() eine lineare Funktion und A ist ein zu bestimmender Faktor.

## Patentansprüche

1. Vorrichtung zur Ermittlung des Kohlendioxidgehalts von Luft mit:
- wenigstens einem kohlendioxidsensitiven Gassensor zur Ausgabe eines Gasmesswerts,
- einer Einrichtung zur Temperatursteuerung des Gassensors,
- einer Einrichtung zur Auswertung des Gasmesswerts zur Ermittlung des Kohlendioxidgehalts der Luft, derart ausgestaltet , dass
- die Einrichtung zur Temperatursteuerung im Betrieb des Gassensors wenigstens zwei verschiedene Temperaturen einstellt,
- bei jeder der zwei verschiedenen Temperaturen wenigstens ein Gasmesswert aufgezeichnet wird,
- die Einrichtung zur Auswertung aus den Gasmesswerten bei den verschiedenen Temperaturen den Kohlendioxidgehalt unter Korrektur des Einflusses der Luftfeuchte ermittelt, indem die Einrichtung zur Auswertung die Gasmesswerte unter der Voraussetzung miteinander verrechnet, dass die Sensitivität des Gassensors auf Kohlendioxid einen anderen Verlauf mit der Temperatur des Gassensors aufweist als die Sensitivität auf Wasser.

2. Vorrichtung gemäß Anspruch 1, bei der der Gassensor ein gassensitives Material (7) aufweist, das primäre Aminogruppen aufweist.

3. Vorrichtung gemäß Anspruch 1 oder 2, wobei der Gassensor ausgestaltet ist, den Gasmesswert durch eine Auswertung der Austrittsarbeit des Materials zu erzeugen.

4. Vorrichtung gemäß einem der vorangehenden Ansprüche, bei der das gassensitive Material (7) ein Polymer aufweist.

5. Vorrichtung gemäß einem der vorangehenden Ansprüche, der-art ausgestaltet, dass eine der verschiedenen Temperaturen eine Temperatur ist, bei der das gassensitive Material (7) nicht auf Kohlendioxid reagiert,

6. Vorrichtung gemäß einem der vorangehenden Ansprüche, bei der der Gassensor einen Feldeffekttransistoraufbau mit einem Luftspalt (6) zwischen dem gassensitiven Material (7) und dem leitenden Kanal des Feldeffekttransistoraufbaus besitzt.

7. Vorrichtung gemäß einem der vorangehenden Ansprüche mit einem Drucksensor.

8. Vorrichtung gemäß einem der vorangehenden Ansprüche mit Mitteln zur Korrektur des Gasmesswerts des Gassensors, derart ausgestaltet, dass der Einfluss der Luftfeuchte auf den Einfluss von Kohlendioxid auf den Gasmesswert korrigiert wird.

9. Vorrichtung gemäß einem der vorangehenden Ansprüche, der-art ausgestaltet, dass die Verweildauer bei der höheren der verschiedenen Temperaturen höchstens der Hälfte der Verweildauer bei der geringeren der verschiedenen Temperaturen entspricht.

10. Vorrichtung gemäß einem der vorangehenden Ansprüche 1 bis 8, derart ausgestaltet, dass die Einstellung der verschiedenen Temperaturen kontinuierlich geschieht, insbesondere indem Temperaturrampen oder sinusartige Temperaturverläufe verwendet werden.

11. Verfahren zur Ermittlung des Kohlendioxidgehalts von Luft mittels eines kohlendioxidsensitiven Gassensors unter Korrektur des Einflusses der Luftfeuchte, bei dem
- im Betrieb der Gassensor auf wenigstens zwei verschiedene Temperaturen gebracht wird,
- bei jeder der zwei verschiedenen Temperaturen wenigstens ein Gasmesswert aufgezeichnet wird,
- aus den Gasmesswerten bei den verschiedenen Temperaturen der Kohlendioxidgehalt unter Korrektur des Einflusses der Luftfeuchte ermittelt wird, indem die Gasmesswerte unter der Voraussetzung miteinander verrechnet werden, dass die Sensitivität des Gassensors auf Kohlendioxid einen anderen Verlauf mit der Temperatur des Gassensors aufweist als die Sensitivität auf Wasser.

## Claims

1. Device for determining the carbon dioxide content of air with:
- at least one carbon dioxide-sensitive gas sensor for output of a measured gas value,
- an apparatus for temperature control of the gas sensor,
- an apparatus for evaluating the measured gas value to determine the carbon dioxide content of the air,
embodied such that
- the apparatus for temperature control sets at least two different temperatures during the operation of the gas sensor,
- at least one measured gas value is recorded at each of the two different temperatures,
- the apparatus for evaluation determines the carbon dioxide content from the measured gas values at the different temperatures while correcting the influence of the air humidity, in that the apparatus for evaluation calculates the measured gas values together, with the assumption that the sensitivity of the gas sensor to carbon dioxide exhibits a different curve with the temperature of the gas sensor than the sensitivity to water.

2. Device according to claim 1, in which the gas sensor has a gas-sensitive material (7) having primary amino groups.

3. Device according to claim 1 or 2, wherein the gas sensor is embodied to create the measured gas value by evaluating the work function of the material.

4. Device according to one of the preceding claims, in which the gas-sensitive material (7) has a polymer.

5. Device according to one of the preceding claims, embodied such that one of the different temperatures is a temperature at which the gas-sensitive material (7) does not react to carbon dioxide.

6. Device according to one of the preceding claims, in which the gas sensor possesses a field effect transistor structure with an air gap (6) between the gas-sensitive material (7) and the conductive channel of the field effect transistor structure.

7. Device according to one of the preceding claims with a pressure sensor.

8. Device according to one of the preceding claims, with means for correcting the measured gas value of the gas sensor, embodied such that the influence of the air humidity is corrected to the influence of carbon dioxide on the measured gas value.

9. Device according to one of the preceding claims, embodied such that the dwell time at the higher of the different temperatures corresponds at the most to half the dwell time at the lower of the different temperatures.

10. Device according to one of the preceding claims 1 to 8, embodied such that the setting of the different temperatures occurs continuously, especially by temperature ramps or sinusoidal temperature curves being used.

11. Method for determining the carbon dioxide content of air by means of a carbon dioxide-sensitive gas sensor while correcting the influence of the air humidity, in which
- at least two different temperatures are reached during the operation of the gas sensor,
- at least one measured gas value is recorded at each of the two different temperatures,
- the carbon dioxide content is determined from the measured gas values at the different temperatures while correcting the influence of the air humidity, by the measured gas values being calculated together, with the assumption that the sensitivity of the gas sensor to carbon dioxide has a different curve with the temperature of the gas sensor than the sensitivity to water.

## Revendications

1. Dispositif de détermination de la teneur en dioxyde de carbone de l'air, comprenant :
- au moins une sonde de gaz sensible au dioxyde de carbone pour l'émission d'une valeur de mesure de gaz,
- un dispositif de commande de la température de la sonde de gaz,
- un dispositif d'exploitation de la valeur de mesure de gaz pour déterminer la teneur en dioxyde de carbone de l'air, conformé de manière à ce que
- le dispositif de commande de la température établit, lorsque la sonde de gaz fonctionne, au moins deux températures différentes,
- on enregistre au moins une valeur de mesure du gaz à chacune des deux températures différentes,
- le dispositif d'exploitation détermine, à partir des valeurs de mesure du gaz aux températures différentes, la teneur en dioxyde de carbone avec correction de l'influence de l'humidité de l'air par le fait que le dispositif d'exploitation compense entre elles les valeurs de mesure du gaz en supposant que la sensibilité de la sonde de gaz au dioxyde de carbone a une courbe en fonction de la température de la sonde de gaz autre que la sensibilité à l'eau.

2. Dispositif suivant la revendication 1, dans lequel la sonde de gaz a un matériau ( 7 ) sensible au gaz, qui a des groupes amino primaires.

3. Dispositif suivant la revendication 1 ou 2, dans lequel la sonde de gaz est constituée pour produire la valeur de mesure du gaz par une exploitation du travail de sortie du matériau.

4. Dispositif suivant l'une des revendications précédentes, dans lequel le matériau ( 7 ) sensible au gaz comporte un polymère.

5. Dispositif suivant l'une des revendications précédentes, conformé de manière à ce que l'une des températures différentes est une température à laquelle le matériau ( 7 ) sensible au gaz ne réagit pas sur le dioxyde de carbone.

6. Dispositif suivant l'une des revendications précédentes, dans lequel la sonde de gaz a une structure de transistor à effet de champ avec un espace ( 6 ) d'air entre le matériau ( 7 ) sensible au gaz et le conduit conducteur de la structure de transistor à effet de champ.

7. Dispositif suivant l'une des revendications précédentes, comprenant une sonde de pression.

8. Dispositif suivant l'une des revendications précédentes, comprenant des moyens de correction de la valeur de mesure du gaz de la sonde de gaz, conformé de manière à corriger, sur la valeur de mesure du gaz, l'influence de l'humidité de l'air sur l'influence du dioxyde de carbonne.

9. Dispositif suivant l'une des revendications précédentes, conformé de manière à ce que la durée de séjour à la plus haute des températures différentes représente au plus la moitié de la durée de séjour à la plus basse des températures différentes.

10. Dispositif suivant l'une des revendications précédentes 1 à 8, conformé de manière à ce que le réglage des températures différentes s'effectue en continue, notamment en utilisant des rampes de température ou des courbes de température sinusoïdales.

11. Procédé de détermination de la teneur en dioxyde de carbone de l'air au moyen d'une sonde de gaz sensible au dioxyde de carbone avec correction de l'influence de l'humidité de l'air dans lequel
- on porte en fonctionnement la sonde de gaz à au moins deux températures différentes,
- on enregistre au moins une valeur de mesure du gaz à chacune des deux températures différentes,
- on détermine, à partir des valeurs de mesure du gaz aux températures différentes, la teneur en dioxyde de carbone avec correction de l'influence de l'humidité de l'air, en comparant entre elles les valeurs de mesure du gaz, en supposant que la sensibilité de la sonde de gaz au dioxyde de carbone a une courbe en fonction de la température de la sonde de gaz autre que la sensibilité à l'eau.
